(19)

Europäisches Patentamt
European Patent Office
Office européen des brevets

(11) **EP 2 720 163 A1**

(12) **EUROPEAN PATENT APPLICATION**

(43) Date of publication:
16.04.2014 Bulletin 2014/16

(51) Int Cl.:
*G06F 19/00* (2011.01)  *C09D 5/08* (2006.01)

(21) Application number: 12188384.7

(22) Date of filing: 12.10.2012

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Steinbeis Advanced Risk Technologies GmbH
70174 Stuttgart (DE)

(72) Inventor: Jovanovic, Aleksandar S.
70569 Stuttgart (DE)

(74) Representative: Patentanwälte
Ruff, Wilhelm, Beier, Dauster & Partner
Kronenstrasse 30
70174 Stuttgart (DE)

(54) **Method for estimating corrosion self-healing and corresponding computer system**

(57) Method for estimating corrosion self-healing and corresponding computer system.

The invention relates to a method for estimating corrosion self-healing of a coating containing inhibition agent particles, and to a computer system configured for use in carrying out this method.

According to the invention the method comprises to perform a corrosion self-healing computer simulation using a DPD model and/or a FE model, wherein input values of corresponding model parameters are input, and wherein the DPD model comprises a particle force modelling containing conservative, dissipative, and random particle-particle interaction forces and an attractive force between each inhibition agent particle and a coated substrate surface, and/or the FE model comprises a diffusion coefficient parameter and a wall binding flux parameter which are adjusted depending on a comparison result of comparing a result of the corrosion self-healing computer simulation with a result of a corresponding corrosion self-healing experiment.

Fig. 7

**Description**

The invention relates to a method for estimating corrosion self-healing of a coating containing inhibition agent particles, and to a computer system configured for use in carrying out this method.

Paints and coatings for industrial and other mechanical parts, such as automotive, aeronautical, and maritime, are designed to reduce corrosion and therefore increase the lifespan of the underlying part. The main methods by which coatings reduce corrosion are by preventing the exposure of the underlying part to oxidizing agents, such as air, and by providing scratch resistance to prevent exposure. Nevertheless, scratches do occur on coated parts and remain on the main contributors to corrosion of parts.

A new class of materials has been recently investigated which looks at the ability of nanomaterials to inhibit corrosion in the presence of a scratch, see e.g. T. Hack et al., Multilevel protection of materials for vehicles by smart nanocontainers, Proc. ASST2009 conference, paper no. 396, 2009. A number of nanocontainers are mixed into the coatings. These nanocontainers are filled with inhibitor molecules, normally a polymer. When a scratch occurs, the nanocontainers shift within the coating and release inhibitor molecules. The nanocontainers and inhibitors create a new layer over the exposed part surface, preventing further corrosion. Numerous studies have been conducted to create and test these new coatings. Current techniques for assessing corrosion damage of parts are time-consuming, expensive, and inefficient. Thus, faster and cheaper methods of assessing corrosion protection are necessary to make these new coating feasible.

A salt fog test is an ISO 9227 standard test examining corrosion resistance of coatings. In the test, a small panel is coated with the coating to be tested and two orthogonal scratches are made on the surface. The panel is placed within a sealed container that is filled with a salt fog. A single test normally takes over 1000 h to complete, after which the panels are removed and any corrosion is examined. Because of the lengthy nature of the test, manufacturers are forced to wait while the process completes, slowing the pace of innovation. Improvements to the salt fog test include running a number of panels through the test in parallel and checking at early time intervals for evidence of corrosion in order to determine poor performance in certain test cases. However, while these improvements assist the manufacturer in optimizing the coating performance, the modifications do not prevent multiple salt fog tests from being necessary.

Computer modelling and simulation is a widely accepted tool for optimizing systems. By using simulations, a manufacturer can virtually run an experiment such as the salt fog test in a matter of minutes at little cost. In such a fashion, an entire optimization could be completed in a fraction of the time required to complete the salt fog tests. A wide variety of simulations techniques is available, but the suitable technique depends on the time-scale and physical-scale. Micro-scale modelling simulates nanometer-scale and smaller objects. In this regime, discrete molecular dynamics are the favoured simulation technique. Molecular dynamics model the interactions between all particles within a system. For very small length-scales, the precision offered by molecular dynamics is important and the number of particles should be small enough to not incur a large computational cost. Macro-scale modelling simulates millimeter-scale and larger objects. Because of the increased computational cost associated with scaling molecular dynamics, continuum models are most often used in macro-scale modelling. Continuum models simulate macroscopic physical rules, such as fluid flow and elasticity, over large length-scales for little computational cost, at the expense of fine detail. In determining corrosion inhibition properties, the nanocontainer-infused material is meso-scale, or micon-scale lengths. Because it is inbetween micro- and macro-regimes, neither molecular dynamics nor full continuum modelling will be sufficient simulation techniques. Dissipative Particle Dynamics (DPD) is a meso-scale modelling technique that models hydrodynamic flow. DPD is a simulation technique based on two different techniques, molecular dynamics and lattice gas automata (LGA), see e.g. P.J. Hoogerbrugge et al., Simulating microscopic hydrodynamic phenomena with dissipative particle dynamics, Europhysics Letters Vol. 19, pp. 155-160, 1992.

Molecular dynamics, as described above, is a powerful tool for simulating random particle movement, but has a non-linearly increasing computational cost as models become larger in scale and longer in time. LGA is a form of particle modelling where each particle is restricted to moving on a lattice. This restriction cuts the computational cost considerably, allowing for meso-scale modelling and emergence of fluid flow laws. However, the restriction causes two flaws in the model: a lack of Galilean invariance and randomness. Galilean invariance is the principle that the laws of physics exist without regard to inertial frame and is an important characteristic of all physical systems.

By combining the two techniques, DPD takes advantage of the benefits of each model while minimizing the flaws. DPD models discrete particles, but does not factor in atomistic properties, instead modelling the movement of the particles based on fluid dynamics and neighbouring particle interactions. The reliance on fluid dynamics and neighbouring interactions instead of atom-atom interactions allows DPD to capture simulated data for longer time scales and larger length scales suitable for meso-scale modelling.

Currently, DPD is used widely in the field of colloid-dispersions, with such applications as lipid modelling, tissue modelling, modelling of movement of drug particles through different parts of the body, see e.g. N. Filipovic et al., Interactions of blood cell constituents: Experimental investigation and computational modelling by discrete particle dynamics algorithm", Microvascular Research, Vol. 75, pp. 279-284, 2008. To the knowledge of the inventors, this is the first time DPD was applied to corrosion-protective material systems, see A. Jovanovic and N. Filipovic, Innovative modelling methods in damage assessment: Application of dissipative particle dynamics to simulation of damage and self-healing of polymer-coated surfaces, Journal of Theoretical and Applied Mechanics, Vol. 44, pp. 637-644, 2006, regarding the similarities between drug particles moving through tissue and inhibitors moving through a coating layer

A high degree of skill and knowledge is required to set up such simulations. Knowledge is needed of modelling techniques and low-level programming languages. Those not skilled in the art may include the manufacturers and researchers of the nanomaterial-infused coatings. While the prior art DPD simulation of damage and self-healing of polymer-coated surfaces was able to provide a first improvement over the lengthy experimental salt fog tests, further improvements are highly desirable in particular concerning the quality of the simulation results as compared to experimental results obtained for real coating systems. In order to make the results of simulation useful to potential end-users, it would also be preferable to have a distribution system whereby end-users could access the data and request specific simulation results based on set parameters.

It is thus the technical problem underlying the invention to provide an improved method for estimating corrosion self-healing using an appropriate computer simulation which yields results in close correspondence to experimental data obtained from real systems, where the method needs significantly less time than the experimental salt fog test and could be a proper basis to make the latter unnecessary. It is also the object of the invention to provide a computer system suited for use in carrying out such method.

The invention solves this problem by providing a method having the features of claim 1, and a computer system according to claim 6.

According to the invention a corrosion self-healing computer simulation is performed which uses a DPD model and/or a finite element (FE) model, wherein input values of corresponding model parameters are input. The DPD model comprises a particle force modelling containing an attractive force between each inhibition agent particle and a coated substrate surface in addition to conservative, dissipative, and random particle-particle interaction forces. The FE model comprises a diffusion coefficient parameter and a wall binding flux parameter which are adjusted depending on a comparison result of comparing a result of the corrosion self-healing computer simulation with a result of a corresponding corrosion self-healing experiment.

It turns out that this method is able to significantly improve the quality of the prior art methods for estimating corrosion self-healing through the use of a computer simulation. In particular, it turns out that using a particle force modelling in the DPD model which takes into account said attractive force between each inhibition agent particle and the coated substrate surface in addition to the particle-particle interaction forces brings the simulation results closer to the results gained from experiments taken at real systems which are modelled by the simulation. The same is true for the FE model including an adjustment of the diffusion coefficient parameter and the wall binding flux parameter depending on comparing the simulation result with an experimental result.

Depending on the needs for the system at hand, the simulation procedure according to the present invention can be based solely on the DPD model, or solely on the FE model, or on a combination of both models. In an advantageous embodiment of the invention at least one DPD model parameter is adjusted depending on said comparison result or, in a combined use of the DPD and the FE models, depending on at least one FE model parameter which is in turn adjusted depending on a comparison of simulation and experimental results. This can further improve the simulation results in order to closely match experimental results.

The invention can be applied to coating systems where the inhibition agent particles are freely dissolved in a primer layer of the coating. Alternatively, the invention can also be applied to coating systems where the inhibition agent particles are included in so-called nanocontainers which are in turn included in a primer layer of the coating.

In an advantageous refinement of the invention the DPD model attractive force is set to be proportional to a difference of a distance of the inhibition agent particle from the coated substrate surface and a maximum length of an attractive domain of the inhibition agent particles. It turns out that this attractive force model is very good suited to describe the inhibition agent particles in typical, real coatings.

In a further development of the invention one or more input values of the following DPD and/or FE model parameters are input when starting the computer simulation: simulation time increment, external force $(F_i^{ext})$, total number of inhibition agent particles in at least two directions, viscosity friction coefficient of primer layer, coefficients of the particle-particle interaction forces, geometrical parameters of primer layer and/or of a primer layer scratch defect and/or of the nanocontainers, percentage of the nanocontainers, nanocontainer filling percentage, diffusion coefficient.

The computer system of the invention is configured for use in carrying out the inventive method. One skilled in the art can easily implement and realize such computer system when considering the information presented in the present application. To this end, the computer system may be equipped with input means for inputting said model parameter input values, and computing means for performing the corrosion self-healing computer simulation. In an embodiment of the invention the computer system may comprise displaying means for displaying output result data of the computer simulation. These data may be data directly obtained from the simulation procedure, or data which are obtained by further processing said direct simulation result data, e.g. by comparing them with experimental data and/or performing a risk analysis.

To give some short, more detailed examples of inventive embodiments here, the method may e.g. be implemented so as to simulate the initial days or weeks of a substrate exposure after a scratch creation. Such scratch may be modelled by a two-dimensional slice of the coating, or by a series of slices such that the scratch runs linearly across the slices to simulate two-and-a-half dimensions. Preferably those method steps which are performed by computation are contained in a program capable of being executed on a processor of the computer system and stored on a computer-readable medium. Inputting of the model parameter input values may be done directly at an input interface of the computer or remote through an internet protocol transmission or other remote protocol transmission. As desired, output result data can be displayed in any usual manner, such as by numbers or by graphical representations such as contour graphs and the like.

In the main the invention will be discussed as being implemented through networked host/client architecture, with transmissions being made between client and host applications. Those skilled in the art should appreciate that the majority of the information processing required to implement the present invention may be completed via a server application, but that a client application is also within the ambit the invention.

Reference throughout this specification will also be made to an end user of the simulation and distribution system being a person associated with a manufacturer of the material coatings. However, it should also be appreciated that the end user of the invention may not necessarily be a person associated with a manufacturer of materials coatings, and can in some instances be researchers or officials who are in a capacity to aid the manufacturer. Those skilled in the art will appreciate that simulation results need not necessarily be used exclusively by a certain subset of users. References to the above only in the main throughout this specification should in no way be seen as limiting.

The invention may provide a statistical result to an end user for a given set of parameters. The result may correspond to a level of corrosion inhibition than can be expected over the lifetime of a salt fog test. Thus, a manufacturer may be able to eliminate certain test series from a salt fog test, saving time and resources. It should also be appreciated that the invention as described above applies dissipative particle dynamics modelling to the simulation of coatings so that it is able to approximate the behaviour of nanocontainers within the coating. As such, a new class of materials will be available for modelling, reducing the time and cost of innovation.

Advantageous embodiments of the invention are described in the following and shown in the drawings in which:

FIG. 1 shows a force diagram illustrating how a particle interacts with neighbouring particles and the various forces as modelled by dissipative particle dynamics,

FIG. 2 shows a schematic of a slice of a nanocontainer-infused material as modelled by the dissipative particle dynamics simulation computer program,

FIG. 3 shows a schematic of how the DPD modelling technique is used to generate a two-and-a-half dimensional profile of the corrosion protection along a crack,

FIG. 4 shows a schematic flowchart of a data analysis from the end of simulation to receipt of information by the end-user,

FIG. 5 shows a contour map as generated by manipulation of data within a spreadsheet,

FIG. 6 shows a model of an end-user interface as displayed on an Internet browser,

FIG. 7 shows a block diagram illustrating a computer system configured to carry out the simulation according to the invention,

FIG. 8 shows a dialogue input parameter interface display for running the DPD model simulation,

FIG. 9 shows a diagram illustrating a current number of inhibitors versus time during a DPD simulation,

FIG. 10 shows a diagram illustrating a percentage of inhibitors covering an exposed surface versus time in a DPD simulation,

FIG. 11 shows a schematic sectional view of a scratch in a metal substrate to be corrosion self-healed through inhibitors included in opened nanocontainers,

FIG. 12 shows a diagram of covering percentage of inhibitors on the substrate surface versus time obtained by FE modelling simulation,

FIG. 13 shows a 3D diagram of the distribution of inhibitors on a scratched surface as obtained through FE modelling simulation,

FIG. 14 shows a 3D diagram of space and time distribution of inhibitors on a scratched surface as obtained by FE modelling simulation.

FIG. 15 shows diagrams related to a comparison of experimental and FE computer simulation fitting results, and

FIG. 16 shows a comparison of experimental and FE model simulation results.

Reference will now be made in detail to the embodiments, examples of which are illustrated in the accompanying drawings, wherein like reference numerals refer to the like elements throughout. The embodiments are described below to explain the invention by referring to the figures.

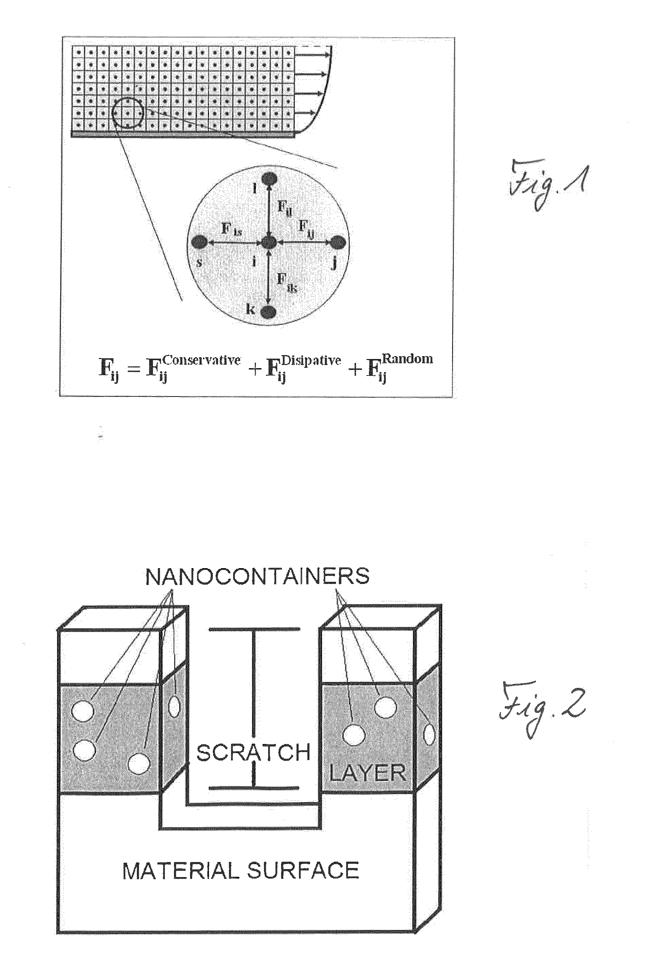

Dissipative particle dynamics (DPD) modelling is used to simulate movement of particles in a system. FIG. 1 displays the various forces between particles and constitutive forces that make up each particle interaction. A DPD model suited for simulating corrosion self-healing is described below. In FIG. 1 particle $i$ has neighbours particles $j$, $k$, $l$, and $s$, each of which is shown to exert a force, $F_{ij}$, $F_{ik}$, $F_{il}$, and $F_{is}$ respectively, on particle i. That force is modelled as the sum of the conservative, dissipative, and random forces, or $F_{ij}^{Total} = F_{ij}^{Conservative} + F_{ij}^{Dissipative} + F_{ij}^{Random}$. This diagram is the basis by which dissipative particle dynamics models the interactions between particles.

In the basic system, i.e. a coating layer with a nanoscopic scratch, N particles occupy a continuous domain of volume V and each particle N has position $r_i$ and velocity $v_i$ where the momentum is continuously updated according to the following equations, with respect to the unit mass:

$$dr_i = v_i dt$$

$$dv_i = \sum_{j \neq i} F_{ij} dt$$

where $\sum_{j \neq i} F_{ij} dt = \sum_{j \neq i} \left( F_{ij}^C dt + F_{ij}^D dt + F_{ij}^R d\sqrt{t} \right)$ and the forces $F^C$, $F^D$ and $F^R$ are conservative, dissipative, and random forces (respectively) that approximate the interactions of particle $j$ on particle $i$. Particle $j$ only interacts with particle $i$ if it is within the radius of influence $r_c$. When it is inside $r_C$, the forces have values of:

$$F_{ij}^C = a_{ij} \left( 1 - \frac{r_{ij}}{r_c} \right) e_{ij}$$

$$F_{ij}^D = -\gamma \left( 1 - \frac{r_{ij}}{r_c} \right)^2 (v_{ij} \cdot e_{ij}) e_{ij}$$

$$F_{ij}^R = \sqrt{2 k_B T \gamma m_i} \left( 1 - \frac{r_{ij}}{r_c} \right) \xi_{ij} e_{ij}$$

Where $a_{ij}$ is the maximum repulsion force per unit mass, $r_{ij}$ is the distance between particles $i$ and $j$, $e_{ij}$ is the unit vector in the direction of $j$ to $i$, $v_{ij} = v_i - v_j$, or the velocity of particle $i$ as observed from the reference frame of particle $j$, $m_i$ is the mass of particle $i$, $\gamma$ is the coefficient of friction, $k_B$ is the Boltzmann constant, $T$ is the equilibrium temperature of the simulation, and $\xi_{ij}$ is a random number with zero mean and unit variance. If $r_{ij} > r_c$, then each of the constitutive forces are assumed to be zero. Examination of the force equations reveals that the magnitude of each force depends on $r_{ij}$, the distance between particles $i$ and $j$. Only the dissipative force depends on the relative velocity $v_{ij}$ of the particles and the direction of each force is given by $e_{ij}$, the unit vector between particles.

The force equations can be reduced using the following dimensionless variables

$$\hat{r}_i = \frac{r_i}{r_c} \quad \hat{r}_{ij} = \frac{r_{ij}}{r_c} \quad \hat{v}_{ij} = \frac{v_{ij}}{v_T} \quad \hat{t} = \frac{t v_T}{r_c}$$

where $v_T = \sqrt{k_B T / m_i}$, or the thermal velocity of the particle $i$. The reductions of the original $r_i$ and $v_i$ equations yield

$$d\hat{r}_i = \hat{v}_i d\hat{t}$$

$$d\hat{v}_i = \sum_{j \neq i} \frac{a_{ij} r_c}{v_T^2}(1 - \hat{r}_{ij}) e_{ij} d\hat{t} - \frac{\gamma r_c}{v_T}(1 - \hat{r}_{ij})^2 (\hat{v}_{ij} \cdot e_{ij}) e_{ij} d\hat{t} + \sqrt{\frac{2\gamma r_c}{v_T}}(1 - \hat{r}_{ij}) \xi_{ij} e_{ij} d\sqrt{\hat{t}}$$

Assuming that all the masses and related repulsion forces ($a_{ij}$) are equal, then the equations above are dependent on two dimensionless numbers:

$$\hat{a} = \frac{a_{ij} r_c}{v_T^2} \qquad \hat{\gamma} = \frac{\gamma r_c}{v_T}$$

Assuming that $v_T$ and $r_c$ do not vary (assuming that the every simulated nanocontainer particle is equivalent), then the values of $a_{ij}$ and $\gamma$ are varied to give the characteristic DPD fluid for simulation.

Further, an external force $F_i^{ext}$ exerted on particle "i" may be considered which usually represents a gradient of pressure or gravity force as a driving force for the fluid domain.

Additionally two dimensionless parameters are used to model correct boundary conditions.

$$\hat{L} = \frac{L}{r_c} \qquad \hat{V_W} = \frac{V_W}{v_T}$$

where L represents the macroscopic size of the system and $V_W$ represents the velocity of the wall.

The particles used in this study represent both inhibition agents and surrounding coating material with different material characteristics. This was achieved by taking into account different repulsion force coefficients $a_{ij}$. The additional interaction forces between particles of inhibition agents, which are placed in the primer layer, and metal substrate particles, are added. These attractive forces are expressed as

$$F_a = k_{sf}\left(1 - \frac{L_{sf}}{L_{sf}^{\max}}\right) \qquad\qquad (7)$$

where $L_{sf}$ is the distance of the inhibition particle from the substrate, $k_{sf}$ is the effective spring constant, and $L_{sf}^{\max}$ is the maximum length of an inhibition particle attractive domain.

The DPD modelling simulates a single two-dimensional slice of the coating system. With reference to FIG. 2, a slice is shown with representative portions of the simulation as described. A void, labelled as a scratch, which exposes an underlying material, labelled as material surface, is present. The material surface is the area that undergoes corrosion and whose protection is modelled. The material surface is coated with a layer. Inside that layer are multiple nanocontainers placed in a random distribution as shown. The major feature of the simulation is the void in the center, labelled as a scratch. The void penetrates from the top of the system through the layer with nanocontainers, exposing the material surface. During simulation, the scratch is filled with a simulated fluid. As the DPD simulation progresses, the nanocontainers accumulate at the material surface and release inhibitors, protecting the material surface against corrosion.

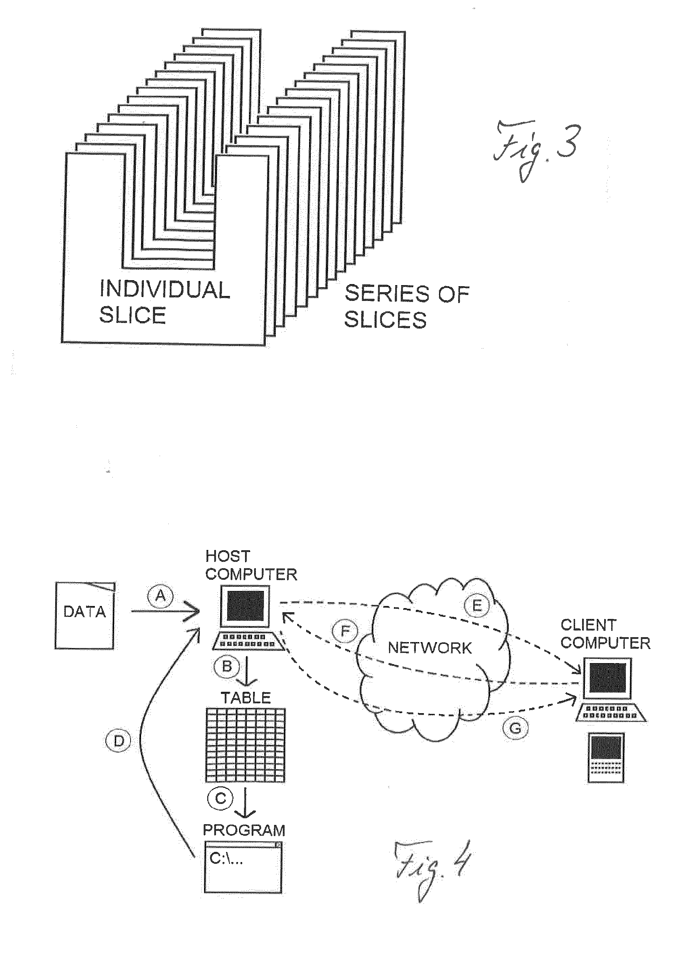

The simulation models the behaviour of the nanocontainers in two-and-a-half dimensions by modelling and stacking a number of slices. With reference to FIG. 3, a series of a given number of individual 11 slices is shown. The scratch void spaces are lined up in a linear fashion such that when viewed from above the scratch or crack is evident. By simulating the series of slices, a profile is generated in the dimension formed by the line, creating the two-and-a-half dimensional profile.

An FE model according to the invention uses a mass transport process for the inhibition agent particle system of the coating governed by the following convection-diffusion equation:

$$\frac{\partial c}{\partial t} + v_x\frac{\partial c}{\partial x} + v_y\frac{\partial c}{\partial y} + v_z\frac{\partial c}{\partial z} = D\left(\frac{\partial^2 c}{\partial x^2} + \frac{\partial^2 c}{\partial y^2} + \frac{\partial^2 c}{\partial z^2}\right) + q^{wall}$$

where c denotes the concentration of inhibitors, i.e. inhibition agent particles; $v_x, v_y$ and $v_z$ are the velocity components in the coordinate system $x,y,z$; $D$ is the diffusion coefficient, assumed to be constant, of the transported material; and $q^{wall}$ is flux of the binding process for inhibitors which adhere on the substrate surface. A similar concept is used for calculation of the volume of inhibitors which are possible to diffuse on the scratch surface. Diffusivity coefficient and wall binding flux are fitting parameters in this FEM model. The drawback of such continuum approach is that particle-particle interaction cannot be modeled but benefit is that a large substrate area is possible to be modeled.

Additional material parameters regarding the physical and temporal dimensions and characteristics of the system can be specified. Changing these parameters does not affect how the simulation works, but will affect the output of the simulation. Additionally, a random seed is used to determine the starting positions of the nanocontainers, ensuring that there is a level of randomness to each simulation. Simulation parameters include, but are not limited to, length of crack, width of crack, height of layer, size of nanocontainers, percentage of nanocontainers in layer (by volume), percentage of nanocontainer filled with inhibitor (by volume), and time length of simulation.

The invention also includes a computer program for carrying out the method steps described above and a distribution system for transferring information to the user and allowing for multi-variable user specification. The computer program embodying the simulation model may be stored on a computer-readable medium where the data could also be stored on computer-readable medium or stored as a downloadable data signal from a network, or could be stored in any other form. Computer-readable medium may include a read only memory (ROM) such as CD ROM or semiconductor ROM, or a magnetic recording medium such as a hard disc. Appropriate hardware for the computer program includes a central processing unit (CPU) for executing the simulation program, a memory for holding temporary data as executed by the CPU, a hard disk for storing the program and the data, a display for showing the information through a graphic user interface (GUI) to the user, and a keyboard and mouse for the user to input the data and manipulate the GUI.

The computer program is run on appropriate hardware and output is stored as a text-based file. This output comprises of a header specifying the value of user-inputted variables for the simulation, a series of time steps, and a series of data points consisting of the simulation result for each of those time steps.

A host computer requires appropriate computer hardware such as a CPU for running the server-side portion of the computer program, a memory for holding temporarily the program, a hard disk for storing the computer program, and a network connection for transferring the information to client computers. The network is comprised of a series of computers with network connections transferring information according to standard Internet protocol. The network connections may be wired or wireless at various parts of the network.

The client computer requires appropriate computer hardware as a CPU for running the software for accessing the web-based computer program, a memory for temporarily holding the information, a hard disk for storing the software for accessing the computer program, a display for showing the GUI to the end-user, a keyboard and mouse for the user to input variables, and a network connection for transferring the information. Appropriate software includes an Internet browser compatible with Javascript.

The computer program consists to two inputs and a series of text which explain the inputs for users moderately-skilled in the art. These inputs correspond to two variables in both the simulated and physical testing. Manipulation of these variables will demonstrate to the end-user the efficacy of a certain variable combination for physical testing. Fig. 4 illustrates a corresponding flowchart including seven steps A to G. In a first step A of the flowchart simulation data is received by the host computer. The data is stored as a text-based file containing raw numeric outputs of percentage of exposed material surface covered by nanocontainers and inhibitors. This data may be transmitted over a network, including as an e-mail attachment over the Internet, as a direct download from a file transfer server, as a peer-to-peer connection such as through a program like Skype, or any other network download platform, or may be transferred using a physical computer readable storage medium, including CD ROM, flash memory, or magnetic strip memory, or any other appropriate medium.. The host computer refers to the computer, or set of computers, which holds and processes the data and is so-called because it stores and hosts the web-based computer program by which the end-user interacts with to obtain information.

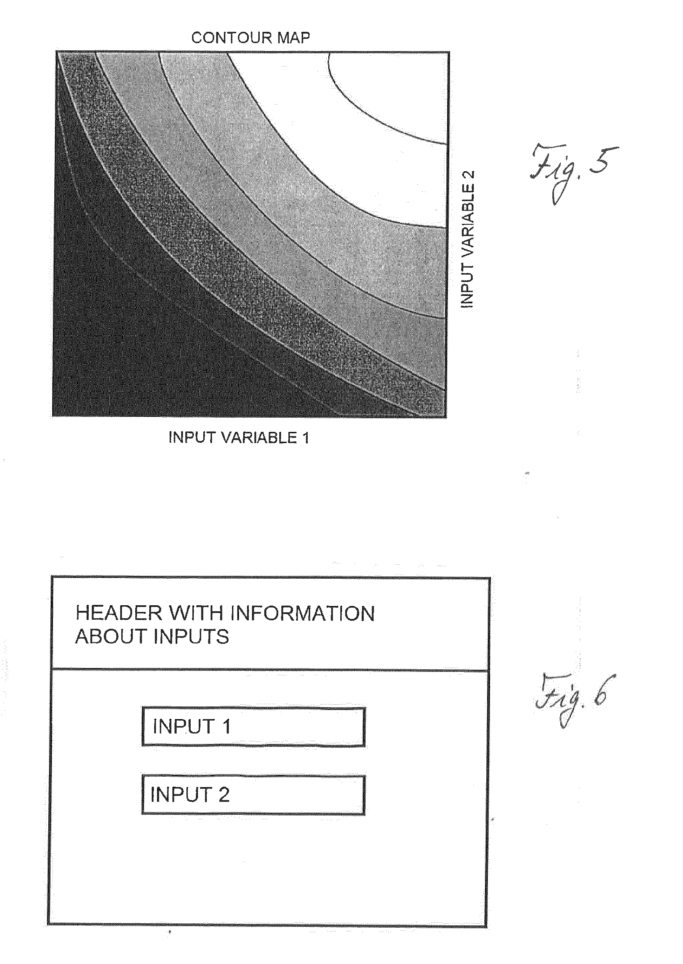

A second step B is the transfer of data to a tabular spreadsheet. This table may be in any spreadsheet program, such as Microsoft® Excel, Libre Calc, or others. For example, the data is analyzed within Microsoft Excel using both human manipulation and Visual Basic for Applications macros. The macros are used to improve the speed of analysis by retabulating data and modifying chart features. These human-based and macro-based manipulations transform the tabular data into a contour map, which correlates simulations outputs for two different variables at a given time within the simulation. The contour map may be color-banded or involve different gray scales to visually show differences in the level of corrosion protection predicted by the simulation, as shown in FIG. 5. The contour map consists of data from a series of simulation runs all at a specific virtual time. The simulations differ by two variables, labelled Input Variable 1 and Input Variable 2. The plot is shaded to display regions of different corrosion protection.

A next step C is the transmission of the contour map and associated information regarding variable specification to the program that is accessed by the end-user. Necessary modifications are performed to ensure that the contour map is properly formatted.

A next step D is the hosting of the program onto the Internet by the host computer. The program is placed inside a website and is usable by anyone with access to that website. A next step E is the access of the web-based computer program by a client computer through a network. The client computer represents an end-user and appropriate browser software. The network is the Internet and transmission of data takes place through standard Internet transmission protocols.

A next step F is the multi-variable input of the end-user through the client computer following the completion of an interface shown in FIG. 6. The web-based computer program has a block of text in the header which explains the interface. Two boxes, labelled Input 1 and Input 2, are displayed for the user to input variables relating to the simulation. Upon submitting the variables, the end-user is shown an appropriately marked contour map, as shown in FIG. 5. The interface thus consists to two inputs and a series of text which explain the inputs for users moderately-skilled in the art. These inputs correspond to two variables in both the simulated and physical testing. Manipulation of these variables will demonstrate to the end-user the efficacy of a certain variable combination for physical testing. In this stage, the variables are transmitted from the client computer to the host computer over the network using standard Internet transmission protocols.

A final step G is the transmission of the contour map to the end-user. Based on the multi-variable input from step F, the appropriate contour map is marked and sent to the client computer over the network using standard Internet transmission protocols.

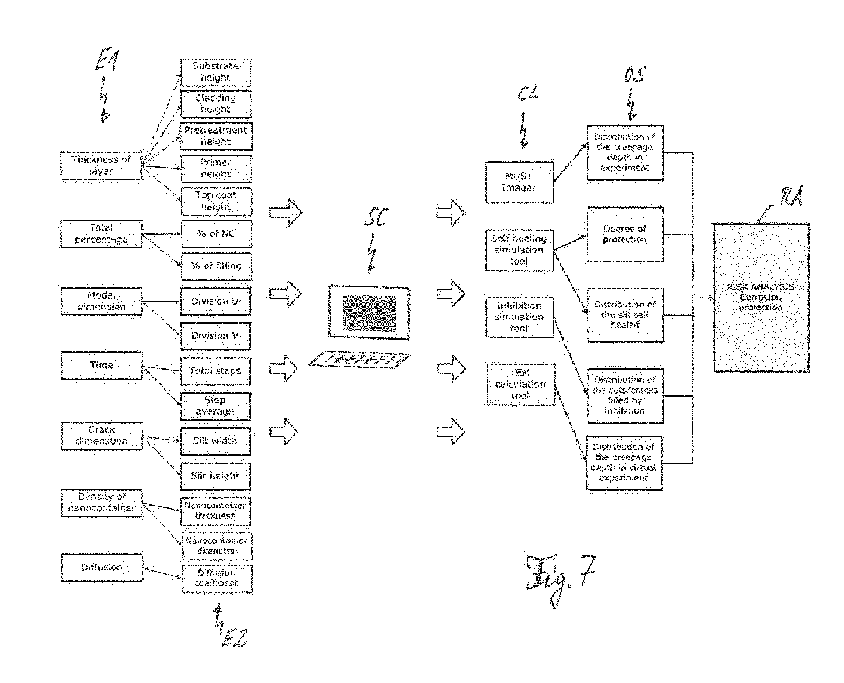

FIG. 7 illustrates a computer system configured to carry out the method of the present invention including the DPD and FE modelling simulation. Proper DPD and FE modelling software is known to the skilled person as such and is implemented through appropriate software and hardware in a simulation computer SC. On an input side the computer SC comprises input means for inputting input values of corresponding DPD and FE modelling parameters such as thickness of coating or primer layer, total percentage of nanocontainers and/or their filling with inhibition agent particles, model dimension, simulation time, scratch or crack dimension, density of nanocontainers, and diffusion parameter, see a first input level E1 in figure 7. Each parameter mentioned above may comprise a plurality of sub-parameters as shown on an input level E2 of figure 7. In the example shown, the layer thickness parameter comprises substrate height, cladding height, pre-treatment height, primer height, and top coat parameters. The total percentage parameter comprises a percentage of nanocontainers and a percentage of filling. The model dimension parameter comprises a division U, i.e. a total number of particles in an X direction at the initial time, and a division V, i.e. a total number of particles in a Y direction orthogonal to the X direction at the initial time. The simulation time parameter includes a number of total simulation steps, i.e. a total number of time steps for the entire modelling period, and a step average, i.e. a total number of steps for writing results for animation. The crack dimension parameter includes geometrical parameters about the scratch or slit, such as slit width and slit height. The nanocontainer density parameter may comprise a nanocontainer thickness and a nanocontainer diameter parameter. The diffusion parameter may include the diffusion coefficient used in the FE modelling.

The simulation computer SC performs the DPD and FE modelling simulation procedures and for this purpose includes the appropriate software and/or hardware tools, such as an imager, an FE calculation tool, a self-healing simulation tool, and an inhibition simulation tool, in particular establishing a corresponding DPD calculation means, see computation level CL in FIG. 7. At an output stage OS the computer system provides for appropriate output means outputting various information useful for the system user. Exemplarily, in FIG. 7 means are illustrated for outputting/displaying the distribution of experimental as well as simulation creepage depth data, a degree of protection, a distribution of the self-healed slit, a distribution of the inhibitor-filled cuts/cracks, a percentage of the crack filled by self-healing inhibitors, and a total number of inhibitors available for protection of the crack. A risk analysis output stage RA of the computer system is configured to perform a risk analysis of the results gained by the method of the invention may be provided, for example to classify in a probability of failure versus consequence of failure diagram a corrosion risk into a plurality of risk levels, such as negligible risk, low risk, medium risk, high risk, and very high risk.

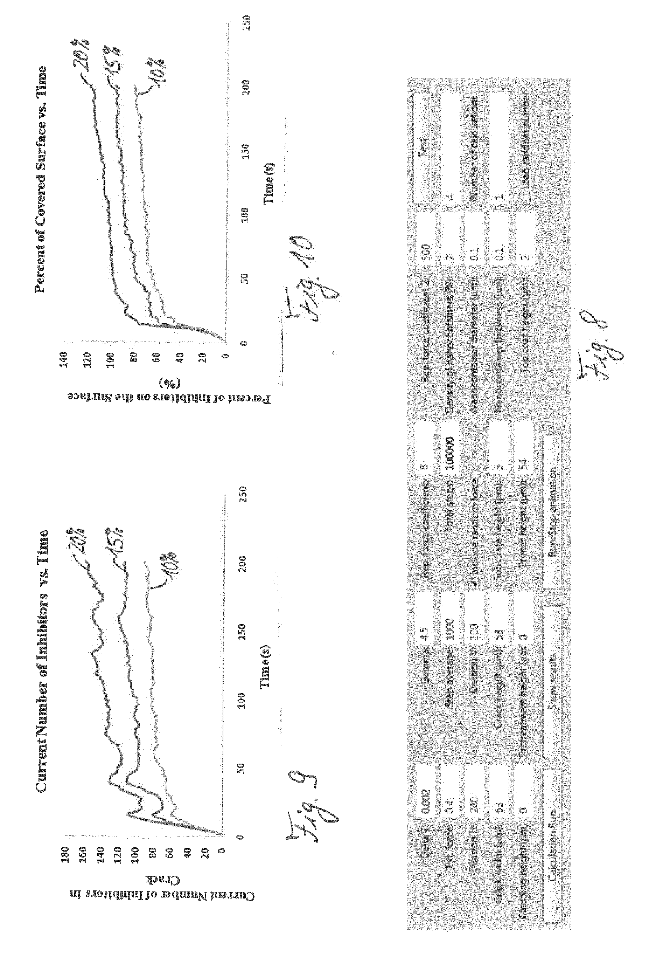

In the following, typical DPD and FE model results obtained by the present invention are presented to further explain and illustrate the invention. In figure 8 a graphic interface for DPD model simulation is shown where input parameters are illustrated which can be changed using a corresponding interface dialogue window of the computer system. In this example the parameters for which input values can be set at the beginning of a DPD modelling simulation are as mentioned before and include in particular the following: "DeltaT" is time step for DPD simulation; "Ext. force" is the external force which acts in Y direction on all particles to produce motion; "Division U" is the total number of particles in X direction at initial time; "Division V" is the total number of particles in Y direction at initial time; "Gamma" is the viscosity friction coefficient used in DPD equations; "Step average" is the total number of steps for writing results for animation; "Total steps" is the total number of time steps for the entire modelling period; "Rep. force. coefficient" is the repulsive force coefficient used for repulsive force for simple DPD particles; "Rep. force. coeff. 2" is the repulsive force coefficient used for the repulsive force between inhibition particles; "Include random force" checking button is used for including/excluding random force in a DPD calculation; and "Density of Nanocontainers" means percent of inhibition particles in the primer layer. A "Calculation" button starts the program execution; A "Run/Stop animation" button is used for start/stop animation which is obtained from the DPD calculated results. A "Show results" button can be activated to display simulation results.

This example is directed to a case having a coating containing a primer layer in which inhibition agent particles, i.e. inhibitors, are dispersed, and the coating is scratched like the case shown in FIG. 2. It is further assumed that water may enter into the scratch.

The external force is assumed to act on all particles. The user can specify a DPD domain by defining a number of particles in X and Y directions. The basic material DPD constants viscosity friction and repulsive force coefficient are also prescribed. An additional repulsive force coefficient is given for inhibition particles in order to keep them close and direct them into the surrounding structural matrix which contains simple DPD particles, like primer and scratch particles. Geometrical parameters of the model include a scratch width, e.g. 0,1 mm, and a nanocontainer diameter, e.g. 100 nm, and a nanocontainer concentration, e.g. 10%, in cases where the inhibitors are confined in nanocontainers. A scaled model was used for simulation due to a large number of particles in the real model.

In a process of primer protection when the scratch occurred, the inhibitors in the primer layer start to interact with the surrounding particles and slip through the scratch on the metal substrate. For pure protection of metal substrate it is necessary to cover the surface with one layer of inhibitors which thickness is about 1nm. During the process of protection, inhibitor particles interact with primer and fluid particles, but with different repulsive force coefficients "Rep. force coefficient" and "Rep. force coefficient 2" in FIG. 8. The additional attractive force Fa was used to connect inhibition with metal substrate particles, so that the inhibitors particles are attached to the walls of the crack.

An exemplary DPD model, with inhibitors in the primer layer with thickness of 4 $\mu$m, consists of a 2D rectangle crack domain with depth of 0.1 mm. Total number of DPD particles was 24000 (240x100). Diameter of nanocontainer was 100 nm and concentration of nanocontainers inside the primer layer was 10%. The total number of time steps for simulation was 100000.

A real scenario of realizing inhibition agents is to assume particle motion inside the crack domain and some surrounded area. Substrate surface coverage and number of inhibitors in the primer were investigated as needed to protect damaged material and repair it completely. The metal coverage with different percent of inhibitors in the primer, namely 10%, 15% and 20%, were tested. The computed results for the current number of inhibitors in the crack and percentage of covered damaged surface after 200s are given in FIG. 9 and FIG. 10, respectively.

As can be seen from FIGS. 9 and 10, different values of inhibitors in the primer give different percentage of covered surface in time. All three different percentages have almost the same rate of the diffusion on the metal substrate while the 20% of inhibitors in the primer give the largest covered surface area.

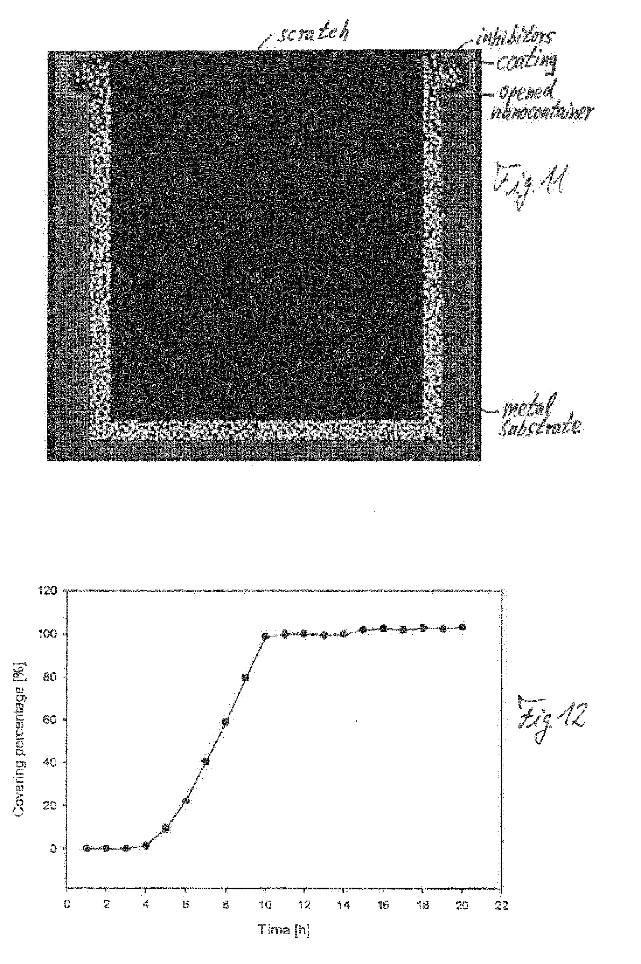

In the case of model with no water existing inside the scratch domain the second DPD model is created as shown in FIG. 11. This model contains nanocontainers in the primer layer, which is scaled due to results presentation. The inhibitors are realized from nanocontainers which are randomize positioned inside the primer domain. The thickness of the primer layer is 400 nm.

The model is matching firstly with the volume needed for cover the scratch surface and time for diffusion of the inhibitors. The production of nanocontainers and inhibitors is quite expensive and it is needed to precisely establish minimum percentage of inhibitors in the nanocontainers for full protection of the treated material.

For the FE modeling the continuum model was defined to consist of a mesh size of 30x30000 = 900000 3D finite elements where inhibitors are randomly prescribed as the influx boundary conditions. In a specific example the scratch dimension is 0.1x100 mm, the primer layer is 4000 nm, nanocontainer diameter is 400 nm, the percentage of inhibitor inside nanocontainers is 20% and the percentage of nanocontainers in the primer or pre-treatment layers is 10%. The convection velocity is assumed to be zero due to the dominant diffusion process. The binding flux was prescribed to be unit which depends on mechanical property of scratch, with no water inclusion on the surface. Computed results during time period of 20h of inhibition process are shown in FIG. 12. It can be seen that almost full 100% coverage is achieved for 10h.

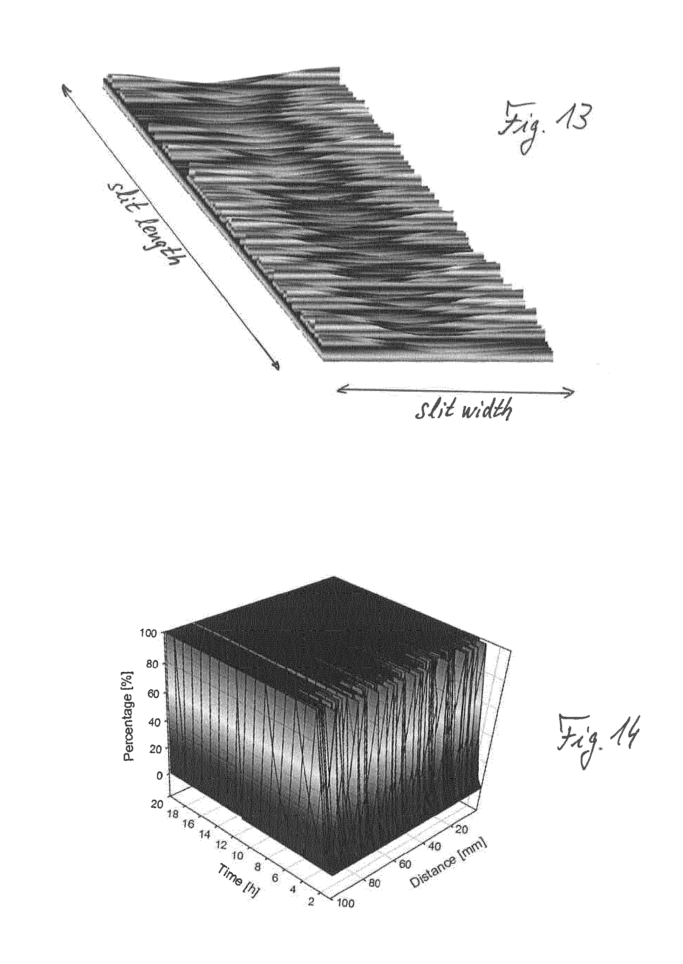

Distribution of inhibitors on the scratch surface for time = 6h is presented in Fig. 13. The width of the scratch is 0.1mm and the length is 100mm in this example. The inhibitors fluxes are randomly distributed along the plate, which cause higher coverage near these plate sides. Space and time distribution of inhibitors on the scratch surface is shown in FIG. 14. It can be seen that after 10h almost all scratch surface is covered with inhibitor layer.

According to an embodiment of the invention one or more of the DPD and FE model parameters are adjusted depending on a comparison of model simulation results and experimental results. A fitting procedure may be used for this purpose. As an example the diffusion coefficient and the diameter of nanocontainers are parameter of the FE model which may be adjusted so that the computer simulations closely match experimental results. A simplex optimization method as described in J. Nelder and R. Mead, A simplex method for function minimization, Computer Journal, 7 (4), pp. 308-313 may be used to reach a best fit. This is a nonlinear procedure which involves only function evaluations, no derivates. The best fit minimizes the sum of squared residuals, a residual being the difference between an experimental value of the creepage distance and the creepage distance provided by a simulation. We have four different experimental creepages defined with 1200 points all together, thus we have 1200 residuals. The sum SE of squared residuals is thus calculated as the sum of the squared difference of the creepage distance $p_i$ for $i$-th point calculated by simulation and the target experimental creepage distance $t_i$ for $i$-th point. A minimum error (SE=3082) was achieved with a diffusion coefficient value being 0.32 $m^2$/s and a diameter of nanocontainers being 0.092 nm.

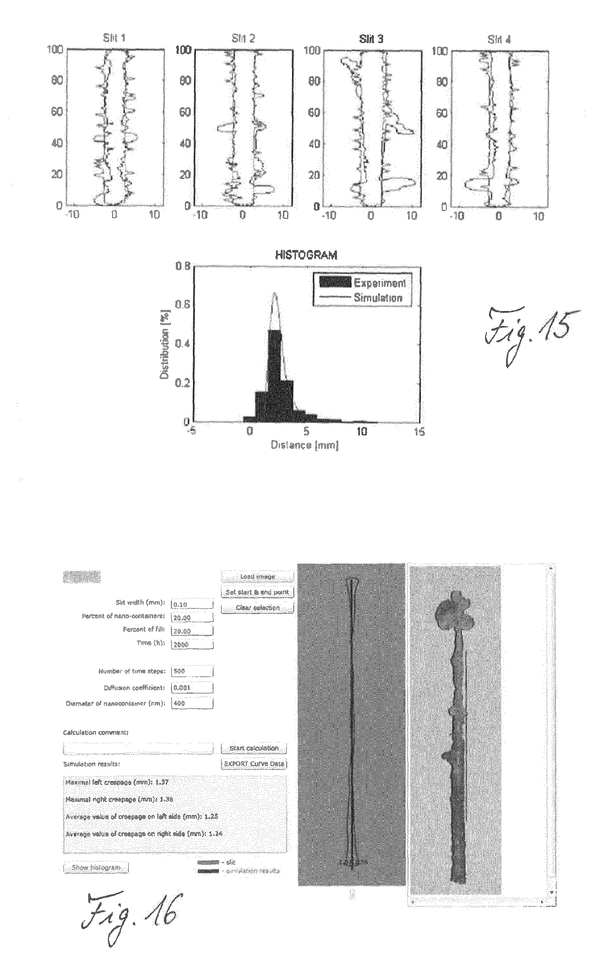

On the top of FIG. 15 are shown four pictures with compared creepage results obtained from simulations and from experimental plates. On the Y axis is given the slit length from 0 to 100 mm. On the X axis are given slit width and creepage values on the left and right side of the slit along them. For calculations different plates from given experimental results were used.

With histogram simulation and experimental results were compared, see lower part of FIG. 15. The Histogram represents frequency of repeating a certain value versus millimeters of obtained creepage.

One page was used to run a calculation on site with parameters which can be seen from FIG. 16. After calculation is finished the results are loaded. Experiment is shown in the right side of FIG. 16. Next to the simulation results are shown. Black color is used for a slit, and grey color for a simulation results - creepage. Through a histogram representation of value frequency versus creepage the simulation and experimental results can be illustrated in comparison.

In corresponding embodiments DPD and FE modeling methods are used in order to simulate self-healing systems with nanocontainers and inhibitors. DPD simulations are reported above as tested on two examples. The first example contains inhibitors inside the primer while the second example simulates realized inhibitors from the nanocontainers which are randomizely distributed inside the primer layer. Three different percentages concentration of inhibitors were used 10%, 15% and 20%. There is a high rate for the covered surface at the beginning of the simulation process while the layer thickness corresponds to the inhibitors concentration in the primer layer. Finite element study revealed that it may be sufficient to have 20% inhibitors in the primer layer with 10% of nanocontainers to completely protect the damaged material. Time for the scratch surface coverage is a model parameter which can be fitted by employing real experimental data. With these two methodologies new hybrid modeling methods are created that integrate fluid dynamics, structural mechanics, chemical reactivity, and phase transitions.

The above description of exemplary embodiments shows that the present invention enables an advantageous estimation of corrosion self-healing using a computer model simulation involving a favourable new type of DPD and/or FE modelling of the corrosion self-healing effect. In addition, advantageous computer interface and display means are provided for this purpose.

**Claims**

1. A method for estimating corrosion self-healing of a coating containing inhibition agent particles, comprising:

   - performing a corrosion self-healing computer simulation using a dissipative particle dynamics (DPD) model and/or a finite element (FE) model, wherein input values of corresponding model parameters are input,
   - wherein the DPD model comprises a particle force modelling containing conservative, dissipative, and random particle-particle interaction forces $(F_{ij}^{C}, F_{ij}^{D}, F_{ij}^{R})$ and an attractive force ($F_a$) between each inhibition

   agent particle and a coated substrate surface, and/or the FE model comprises a diffusion coefficient parameter and a wall binding flux parameter which are adjusted depending on a comparison result of comparing a result of the corrosion self-healing computer simulation with a result of a corresponding corrosion self-healing experiment.

2. The method of claim 1, wherein at least one DPD model parameter is adjusted depending on said comparison result or depending on at least one FE model parameter adjusted depending on said comparison.

3. The method of claim 1 or 2, wherein the coating is defined to include nanocontainers in a primer layer, said nanocontainers including said inhibition agent particles.

4. The method according to any of claims 1 to 3, wherein said DPD model attractive force ($F_a$) is set to be proportional to a difference of a distance ($L_{sf}$) of the inhibition agent particle from the coated substrate surface and a maximum

   length $(L_{sf}^{\max})$ of an attractive domain of the inhibition agent particles.

5. The method according to any of claims 1 to 4, wherein said input values comprise at least one input value of any of the following DPD and/or FE model parameters: simulation time increment, external force $(F_i^{ext})$, total number

   of inhibition agent particles in at least two directions, viscosity friction coefficient of primer layer, coefficients of the particle-particle interaction forces, geometrical parameters of primer layer and/or of a primer layer scratch defect and/or of the nanocontainers, percentage of the nanocontainers, nanocontainer filling percentage, diffusion coefficient.

6. Computer system, **characterized in that** it is configured for use in carrying out the method according to any of claims 1 to 5.

7. Computer system according to claim 6, comprising input means for inputting said input values of the model parameters and computing means for performing said corrosion self-healing computer simulation.

8. Computer system according to claim 6 or 7, comprising display means for displaying output result data of the computer simulation.

$$\mathbf{F}_{ij} = \mathbf{F}_{ij}^{\text{Conservative}} + \mathbf{F}_{ij}^{\text{Disipative}} + \mathbf{F}_{ij}^{\text{Random}}$$

Fig.1

NANOCONTAINERS

SCRATCH

LAYER

MATERIAL SURFACE

Fig.2

INDIVIDUAL
SLICE

SERIES OF
SLICES

Fig. 3

DATA

HOST
COMPUTER

(A)

(B)

TABLE

(C)

PROGRAM

C:\...

(D)

NETWORK

(E)

(F)

(G)

CLIENT
COMPUTER

Fig. 4

CONTOUR MAP

INPUT VARIABLE 2

INPUT VARIABLE 1

*Fig. 5*

HEADER WITH INFORMATION
ABOUT INPUTS

INPUT 1

INPUT 2

*Fig. 6*

E1

| Thickness of layer | → Substrate height |
| | → Cladding height |
| | → Pretreatment height |
| | → Primer height |
| | → Top coat height |

| Total percentage | → % of NC |
| | → % of filling |

| Model dimension | → Division U |
| | → Division V |

| Time | → Total steps |
| | → Step average |

| Crack dimension | → Slit width |
| | → Slit height |

| Density of nanocontainer | → Nanocontainer thickness |
| | → Nanocontainer diameter |

| Diffusion | → Diffusion coefficient |

E2

SC

CL     OS

| MUST Imager | → Distribution of the creepage depth in experiment |
| Self healing simulation tool | → Degree of protection |
| Inhibition simulation tool | → Distribution of the slit self healed |
| FEM calculation tool | → Distribution of the cuts/cracks filled by inhibition |
| | → Distribution of the creepage depth in virtual experiment |

RA

RISK ANALYSIS
Corrosion protection

*Fig. 7*

## Current Number of Inhibitors vs. Time

*Fig. 9*

## Percent of Covered Surface vs. Time

*Fig. 10*

| | | | | | |
|---|---|---|---|---|---|
| Delta T: 0.002 | Gamma: 4.5 | Rep. force coefficient: 8 | Rep. force coefficient 2: 500 | | Test |
| Ext. force: 0.4 | Step average: 1000 | Total steps: 100000 | Density of nanocontainers (%): 2 | 4 | |
| Division U: 240 | Division V: 100 | ☑ Include random force | Nanocontainer diameter (μm): 0.1 | Number of calculations | |
| Crack width (μm): 63 | Crack height (μm): 58 | Substrate height (μm): 5 | Nanocontainer thickness (μm): 0.1 | 1 | |
| Cladding height (μm): 0 | Pretreatment height (μm: 0 | Primer height (μm): 54 | Top coat height (μm): 2 | ☐ Load random number | |
| Calculation Run | Show results | Run/Stop animation | | | |

*Fig. 8*

scratch

inhibitors

coating

opened nanocontainer

*Fig. 11*

metal substrate

*Fig. 12*

Fig. 13

Fig. 14

Fig.15

Fig.16

Europäisches
Patentamt

European
Patent Office

Office européen
des brevets

# EUROPEAN SEARCH REPORT

Application Number

EP 12 18 8384

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FILIPOVIC N. ET AL: "MODELING OF SELF-HEALING MATERIALS USING NANOCONTAINERS", CONTEMPORARY MATERIALS, vol. 2, no. 1, 1 January 2011 (2011-01-01) , pages 18-26, XP055061317, ISSN: 1986-8669, DOI: 10.5767/anurs.cmat.110201.en.018F * the whole document * * in particular: * * paragraph [001.] - paragraph [2.3.] * * figures 1,2,7-12 * | 1-8 | INV. G06F19/00 ADD. C09D5/08 |
| A | JOVANOVIC A. S. ET AL: "Innovative Modelling Methods in Damage Assessment: Application of Dissipative Particle Dynamics to Simulation of Damage and Self-Healing of Polymer-Coated Surfaces", JOURNAL OF THEORETICAL AND APPLIED MECHANICS, vol. 44, no. 3, 2006, pages 637-648, XP008161798, * abstract * * page 639, lines 15-30 * * page 643, lines 7-20 * * figures 5,6 * | 1-8 | |

| | | | TECHNICAL FIELDS SEARCHED (IPC) |
|---|---|---|---|
| | | | G06F C09D |

The present search report has been drawn up for all claims

| Place of search | Date of completion of the search | Examiner |
|---|---|---|
| The Hague | 29 April 2013 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. HACK et al.** Multilevel protection of materials for vehicles by smart nanocontainers. *Proc. ASST2009 conference,* 2009
- **P.J. HOOGERBRUGGE et al.** Simulating microscopic hydrodynamic phenomena with dissipative particle dynamics. *Europhysics Letters,* 1992, vol. 19, 155-160
- **N. FILIPOVIC et al.** Interactions of blood cell constituents: Experimental investigation and computational modelling by discrete particle dynamics algorithm. *Microvascular Research,* 2008, vol. 75, 279-284
- **A. JOVANOVIC ; N. FILIPOVIC.** Innovative modelling methods in damage assessment: Application of dissipative particle dynamics to simulation of damage and self-healing of polymer-coated surfaces. *Journal of Theoretical and Applied Mechanics,* 2006, vol. 44, 637-644
- **J. NELDER ; R. MEAD.** A simplex method for function minimization. *Computer Journal,* vol. 7 (4), 308-313